# EUROPEAN PATENT APPLICATION

(11) **EP 4 375 302 A1**
(43) Date of publication of application: **29.05.2024**
(21) Application number: 22845944.2
(22) Date of filing: 20.07.2022
(51) Int. Cl.: C08F 2/44, C07C 323/52, C08G 59/66, C08G 75/045

(54) **THIOL-CONTAINING COMPOSITION, PHOTOCURABLE COMPOSITION, AND THERMOSETTING COMPOSITION**

(30) Priority: 20.07.2021 JP 2021119746
(71) Applicant: Resonac Corporation, Tokyo 105-7325 (JP)
(72) Inventor: HASHIMOTO Yuji, Tokyo 105-8518 (JP); NISHIMURA Norihito, Tokyo 105-8518 (JP)
(74) Representative: Strehl Schübel-Hopf & Partner
(86) International application number: PCT/JP2022/028178
(87) International publication number: WO 2023/003011

(57) **Abstract**

A thiol-containing composition includes polyfunctional secondary thiol compounds (A) and disulfide dimers (B), wherein the polyfunctional secondary thiol compound (A) are each an ester of a polyhydric alcohol with a carboxylic acid having a secondary mercapto group; the disulfide dimers (B) are disulfide dimers of the polyfunctional secondary thiol compounds (A); and the amount of the disulfide dimers (B) is 0.001 to 1.5 parts by mass based on 100 parts by mass of the polyfunctional secondary thiol compounds (A).

## Description

### [Technical Field]

The present invention relates to a thiol-containing composition, and a photosetting composition and a thermosetting composition which contain the thiol-containing composition.

Priority is claimed on Japanese Patent Application No. 2021-119746, filed July 20, 2021, the content of which is incorporated herein by reference.

### [Background Art]

A polyfunctional thiol compound having two or more mercapto groups in the molecule is mixed with a compound having an ethylenically unsaturated group or with a compound having an epoxy group to be used as materials for curable compositions (for example, refer to Patent Documents 1 to 4). Such curable compositions are widely used in applications such as adhesives, coating materials, encapsulants, and solder resist.

### [Citation List]

### [Patent Documents]

[Patent Document 1]
   Japanese Unexamined Patent Application, First Publication No. 2020-164562
[Patent Document 2]
   Japanese Unexamined Patent Application, First Publication No. 2020-105464
[Patent Document 3]
   Japanese Unexamined Patent Application, First Publication No. 2016-60839
[Patent Document 4]
   Japanese Unexamined Patent Application, First Publication No. 2014-1291
[Patent Document 5]
   Japanese Unexamined Patent Application, First Publication No. 2011-084479
[Patent Document 6]
   Japanese Patent No. 4911666
[Patent Document 7]
   Japanese Patent No. 4917294

### [Non-Patent Document]

[Non-Patent Document 1]
   J. Am. Chem. Soc. 1982, 104, 22, 6045-6053
[Non-Patent Document 2]
   J. Am. Chem. Soc. 1988, 110, 23, 7813-7827

### [Summary of the Invention]

### [Technical Problem]

Among polyfunctional thiol compounds, thiol-containing compositions containing secondary thiols have excellent storage stability compared to thiol-containing compositions containing primary thiols.

However, even in thiol-containing compositions containing secondary thiols, sufficient storage stability may not be obtained in some cases, and further improvement in storage stability is required.

In addition, even in curable compositions containing thiol-containing compositions, sufficient storage stability may not be obtained in some cases, and further improvement in storage stability is required.

The present invention has been made in consideration of the above-described circumstances, and an object of the present invention is to provide a thiol-containing composition having excellent storage stability.

In addition, another object of the present invention is to provide a photosetting composition and a thermosetting composition which contain the thiol-containing composition of the present invention and have excellent storage stability.

### [Solution to Problem]

A first aspect of the present invention provides the following thiol-containing composition.
[1] A thiol-containing composition including: polyfunctional secondary thiol compounds (A); and disulfide dimers (B), in which the polyfunctional secondary thiol compounds (A) are each an ester of a polyhydric alcohol with a carboxylic acid having a secondary mercapto group, the disulfide dimers (B) are disulfide dimers of the polyfunctional secondary thiol compounds (A), and the amount of disulfide dimers (B) is 0.001 to 1.5 parts by mass based on 100 parts by mass of the polyfunctional secondary thiol compounds (A).

The thiol-containing composition of the first aspect of the present invention preferably has features described in [2] to [4] below. It is also preferable to arbitrarily combine two or more of the features described in [2] to [4] below.

[2] The thiol-containing composition according to [1], in which the amount of disulfide dimers (B) is 0.001 to 1.0 parts by mass based on 100 parts by mass of the polyfunctional secondary thiol compounds (A).

[3] The thiol-containing composition according to [1] or [2], in which the polyfunctional secondary thiol compounds (A) are each an ester of 3-mercaptobutanoic acid with a polyhydric alcohol.

[4] The thiol-containing composition according to any one of [1] to [3], in which the polyfunctional secondary thiol compounds (A) are each at least one selected from Formulae (1) to (4) below.

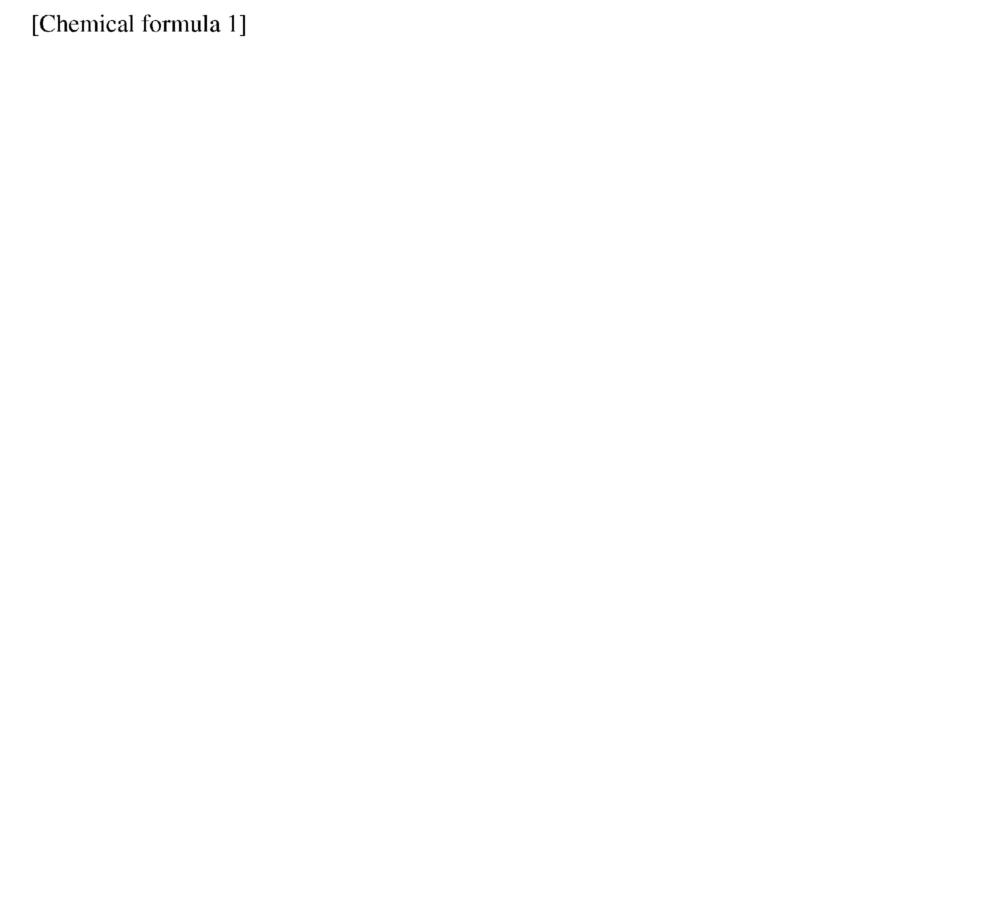

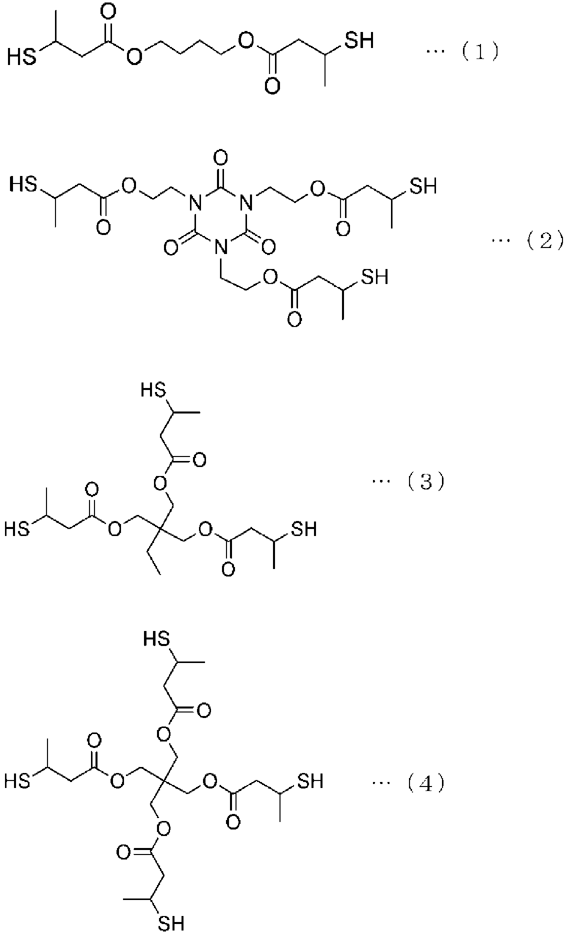

A second aspect of the present invention provides the following photosetting composition.

[5] A photosetting composition including: the thiol-containing composition according to any one of [1] to [4]; a compound having an ethylenically unsaturated group; and a photopolymerization initiator.

The photosetting composition of the second aspect of the present invention preferably has features described in [6] or [7] below. It is also preferable to combine the features described in [6] or [7] below.

[6] The photosetting composition according to [5], in which the amount of the thiol-containing composition is 1 to 200 parts by mass based on 100 parts by mass of the compound having an ethylenically unsaturated group.

[7] The photosetting composition according to [5] of [6], in which the amount of the photopolymerization initiator is 1 to 100 parts by mass based on 100 parts by mass of the compound having an ethylenically unsaturated group.

A third aspect of the present invention provides the following thermosetting composition.

[8] A thermosetting composition including: the thiol-containing composition according to any one of [1] to [4]; a compound having an epoxy group; and a curing catalyst.

The curable composition of the third aspect of the present invention preferably has features described in [9] of [10] below. It is also preferable to combine the features described in [9] or [10] below.

[9] The thermosetting composition according to [8], in which the amount of the thiol-containing composition is 1 to 200 parts by mass based on 100 parts by mass of the compound having an epoxy group.

[10] The thermosetting composition according to [8] or [9], in which the amount of the curing catalyst is 0.05 to 10 parts by mass based on 100 parts by mass of the compound having an epoxy group.

### [Advantageous Effects of Invention]

The thiol-containing composition of the present invention includes: polyfunctional secondary thiol compounds (A) each of which is an ester of a polyhydric alcohol with a carboxylic acid having a secondary mercapto group; and disulfide dimers (B) of the polyfunctional secondary thiol compounds (A), in which the amount of the disulfide dimers (B) is 0.001 to 1.5 parts by mass based on 100 parts by mass of the polyfunctional secondary thiol compounds (A). For this reason, the thiol-containing composition of the present invention has sufficient features and performance due to incorporation of the polyfunctional secondary thiol compounds (A), and has superior storage stability compared to the polyfunctional secondary thiol compounds (A).

The photosetting composition and thermosetting composition of the present invention contains the thiol-containing composition of the present invention. For this reason, the features and performance due to incorporation of the polyfunctional secondary thiol compounds (A) can be sufficiently obtained. Moreover, the photosetting composition and thermosetting composition of the present invention have superior storage stability compared to a case where the polyfunctional secondary thiol compounds (A) are used instead of the thiol-containing composition.

### [Description of Embodiment]

The present inventors have conducted extensive studies to solve the above-described problems.

As a result, it has been found that, by making a thiol-containing composition containing polyfunctional secondary thiol compounds (A) each of which is an ester of a polyhydric alcohol with a carboxylic acid having a secondary mercapto group and disulfide dimers (B) of the polyfunctional secondary thiol compounds (A), decomposition of the polyfunctional secondary thiol compounds (A) can be suppressed and storage stability of the thiol-containing composition and curable compositions (a photosetting composition and a thermosetting composition) containing this thiol-containing composition is improved.

It is assumed that the decomposition of the polyfunctional secondary thiol compounds (A) contained in the above-described thiol-containing composition is suppressed by the mechanism shown below.

Disulfide compounds have a property of capturing radicals. For this reason, in the thiol-containing composition containing the polyfunctional secondary thiol compounds (A) and the disulfide dimers (B), even if radicals are generated by light or heat, they are inactivated by the disulfide dimers (B).

In addition, disulfide compounds generate thiyl radicals when disulfide bonds are cleaved by light. Since thiyl radicals have relatively low reactivity, they do not promote decomposition of the polyfunctional secondary thiol compounds (A). On the other hand, thiyl radicals preferentially react with highly reactive radicals generated by light or heat.

Accordingly, it is assumed that the chain decomposition reaction for the polyfunctional secondary thiol compounds (A) caused by radicals is suppressed in the above-described thiol-containing composition.

The present inventors have further conducted extensive studies. They have confirmed that the thiol-containing composition containing 0.001 to 1.5 parts by mass of the disulfide dimers (B) of the polyfunctional secondary thiol compounds (A) based on 100 parts by mass of the polyfunctional secondary thiol compounds (A), and the curable composition containing this thiol-containing composition have sufficient features and performance due to incorporation of the polyfunctional secondary thiol compounds (A) and have favorable storage stability, thus leading to realization of the present invention.

Hereinafter, preferred examples of the thiol-containing composition, photosetting composition, and thermosetting composition of the present invention will be described in detail. The present invention is not limited to only the embodiment shown below. Numbers, amount, types, positions, ratios, materials, configurations, and the like in the present invention can be added, omitted, replaced, or modified within the scope not departing from the gist of the present invention.

### [Thiol-containing composition]

A thiol-containing composition of the present embodiment contains polyfunctional secondary thiol compounds (A) and disulfide dimers (B) of the polyfunctional secondary thiol compounds (A).

### "Polyfunctional secondary thiol compounds (A)"

A polyfunctional secondary thiol compound (A) contained in the thiol-containing composition of the present embodiment is an ester of a polyhydric alcohol with a carboxylic acid having a secondary mercapto group. The polyfunctional secondary thiol compound (A) may be used alone or in combination of two or more thereof. Examples of carboxylic acids having a secondary mercapto group include 2-mercaptopropionic acid, 2-mercaptobutanoic acid, and 3-mercaptobutanoic acid.

Examples of polyhydric alcohols include 1,4-butanediol, 1,3,5-tris(2-hydroxyethyl)-1,3,5-triazine-2,4,6(1H,3H,5H)-trione, trimethylolpropane, and pentaerythritol.

The polyhydric alcohol may be an alcohol containing two or more alcoholic hydroxyl groups and having a bisphenol skeleton or a fluorene skeleton. Examples of alcohols having a bisphenol skeleton include compounds obtained by adding alkylene oxides such as ethylene oxide and propylene oxide to phenolic hydroxyl groups such as 2,2-bis(4-hydroxyphenyl)propane and 2,2-bis(4-hydroxy-3,5-dimethylphenyl)propane. Examples of alcohols having a fluorene skeleton include compounds obtained by adding alkylene oxides such as ethylene oxide and propylene oxide to phenolic hydroxyl groups such as 9,9-bis(4-hydroxyphenyl)fluorene.

The polyfunctional secondary thiol compound (A) is preferably an ester of 3-mercaptobutanoic acid with a polyhydric alcohol (3-mercaptobutanoic acid ester) for the reasons shown below. That is, 3-mercaptobutanoic acid has a simple structure and is one of the most easily available compounds among carboxylic acids having a secondary mercapto group. In addition, polyhydric alcohols with various structures can be obtained relatively easily. Thus, in a case where the polyfunctional secondary thiol compound (A) is 3-mercaptobutanoic acid ester, a polyfunctional secondary thiol compound (A) having various numbers of functional groups and skeleton structures can be obtained relatively easily.

Examples of 3-mercaptobutanoic acid ester include 1,4-bis(3-mercaptobutyryloxy)butane represented by Formula (1) below, 1,3,5-tris(2-(3-sulfanylbutanoyloxy)ethyl)-1,3,5-triazinane-2,4,6-trione represented by Formula (2) below, trimethylolpropane tris(3-mercaptobutyrate) represented by Formula (3) below, pentaerythritol tetrakis(3-mercaptobutyrate) represented by Formula (4) below, 3-mercaptobutanoic acid ester which is represented by General Formula (5) below and has a bisphenol A structure, and 3-mercaptobutanoic acid ester which is represented by General Formula (6) below and has a fluorene structure.

The polyfunctional secondary thiol compound (A) is preferably 3-mercaptobutanoic acid ester represented by Formulae (1) to (4) below because a thiol-containing composition that can be suitably used as various raw materials for thermosetting compositions and photosetting compositions is obtained. 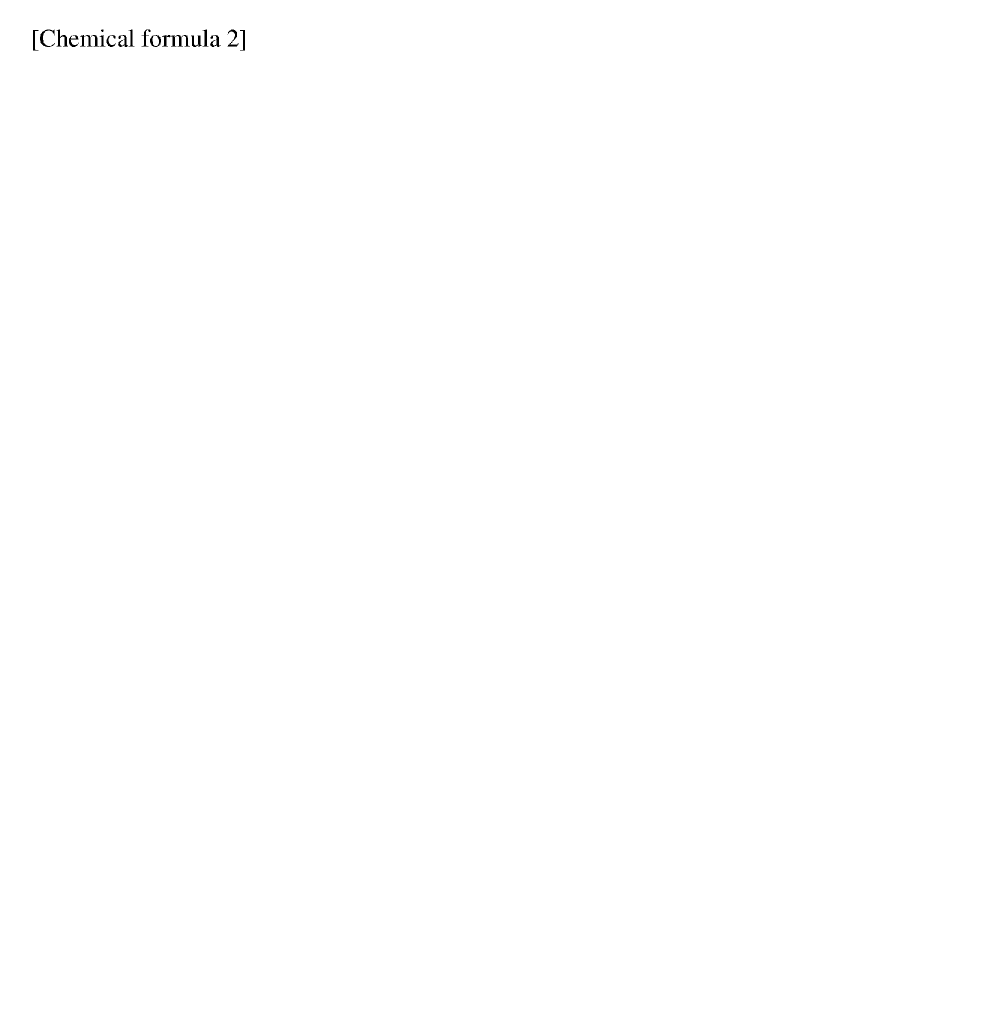 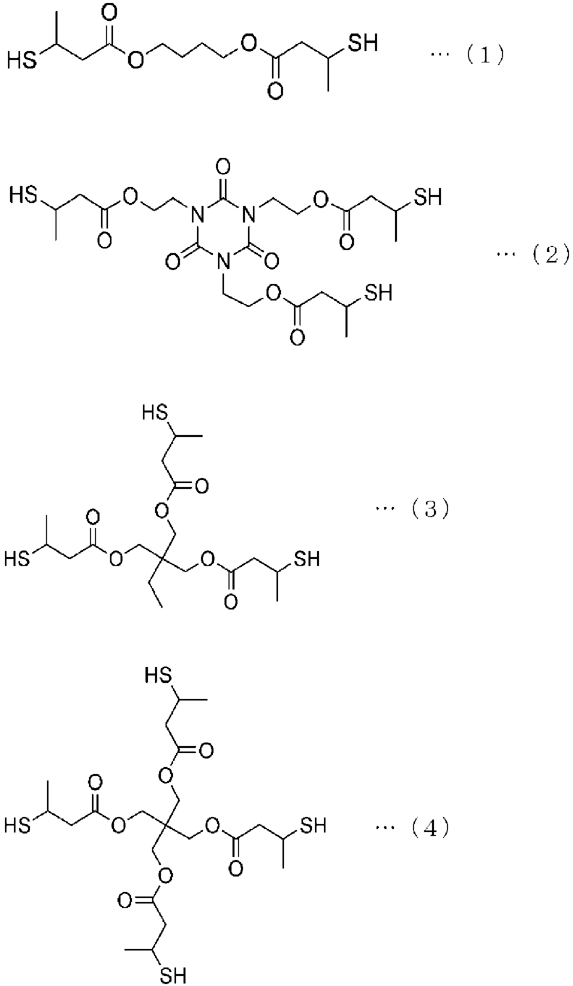 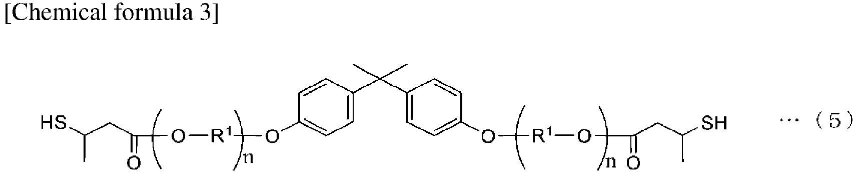 (In Formula (5), R¹ represents a linear or branched alkylene group having 2 to 6 carbon atoms; and n represents an integer of 1 to 4.) 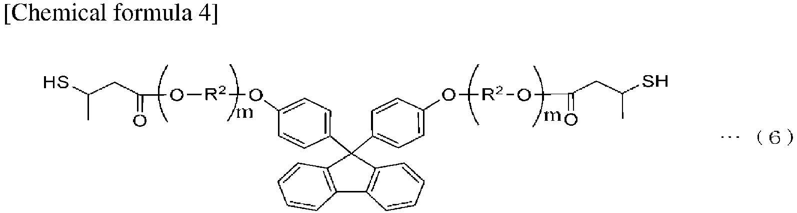 (In Formula (6), R² represents a linear or branched alkylene group having 2 to 6 carbon atoms; and m represents an integer of 1 to 4.)

In the 3-mercaptobutanoic acid ester represented by General Formula (5), R¹ represents a linear or branched alkylene group having 2 to 6 carbon atoms. Specific examples of alkylene groups represented by R¹ include -CH₂CH₂-, -CH(CH₃)CH₂-, and - CH₂CH(CH₃)-, and -CH₂CH₂- is preferable.

In addition, in the 3-mercaptobutanoic acid ester represented by General Formula (5), n represents an integer of 1 to 4, and 1 is preferable.

Specific examples of 3-mercaptobutanoic acid ester represented by General Formula (5) include 2,2-bis{4-(3-mercaptobutyroyloxyethoxy)phenyl}propane (R¹ in Formula (5) is -CH₂CH₂-, and n is 1).

The polyfunctional secondary thiol compound (A) is preferably 3-mercaptobutanoic acid ester represented by General Formula (5) because a photosetting composition with excellent development margin can be provided using a thiol-containing composition containing this compound as a raw material.

In the 3-mercaptobutanoic acid ester represented by General Formula (6), R² represents a linear or branched alkylene group having 2 to 6 carbon atoms. Specific examples of alkylene groups represented by R² include -CH₂CH₂-, -CH(CH₃)CH₂-, and - CH₂CH(CH₃)-, and -CH₂CH₂- is preferable.

In addition, in the 3-mercaptobutanoic acid ester represented by General Formula (6), m represents an integer of 1 to 4, and 1 is preferable.

Specific examples of 3-mercaptobutanoic acid ester represented by General Formula (6) include 9,9-bis{4-(3-mercaptobutyroyloxyethoxy)phenyl}fluorene (R² in Formula (6) is -CH₂CH₂-, and m is 1).

The polyfunctional secondary thiol compound (A) is preferably 3-mercaptobutanoic acid ester represented by General Formula (6) because a photosetting composition with excellent development margin can be provided using a thiol-containing composition containing this compound as a raw material.

The polyfunctional secondary thiol compound (A) can be produced through a well-known conventional method.

For example, in a case where the polyfunctional secondary thiol compound (A) is 3-mercaptobutanoic acid ester, it can be produced through a method of causing an esterification reaction between 3-mercaptobutanoic acid and a polyhydric alcohol.

The 3-mercaptobutanoic acid ester represented by Formulae (1) to (4) above can be synthesized, for example, through a method disclosed in Patent Document 5. The 3-mercaptobutanoic acid ester represented by General Formula (5) can be synthesized, for example, through a method disclosed in Patent Document 6. The 3-mercaptobutanoic acid ester represented by General Formula (6) can be synthesized, for example, through a method disclosed in Patent Document 7.

### "Disulfide dimers (B)"

Disulfide dimers (B) contained in the thiol-containing composition of the present embodiment suppress decomposition of the polyfunctional secondary thiol compounds (A) contained in the thiol-containing composition. The disulfide dimers (B) are disulfide dimers (B) of the polyfunctional secondary thiol compounds (A) contained in the thiol-containing composition, and have a molecular structure in which two molecules of the same polyfunctional secondary thiol compounds (A) are disulfide-bonded.

That is, a disulfide dimer (B) contained in the thiol-containing composition of the present embodiment is a compound in which a pair of mercapto groups selected one by one from each of the two molecules of the same polyfunctional secondary thiol compounds (A) contained in the thiol-containing composition are linked by forming a disulfide bond.

In other words, in a case where one mercapto group contained in the structure of a polyfunctional secondary thiol compound (A) is clearly indicated, and the polyfunctional secondary thiol compound (A) contained in the thiol-containing composition is represented by a chemical formula RSH (which is a structure in which R is any group and one -SH contained in the polyfunctional secondary thiol compound (A) is excluded), the disulfide dimer (B) Is a compound represented by a chemical formula RSSR.

In a case where the polyfunctional secondary thiol compound (A) has an asymmetric structure, multiple types of disulfide dimers (B) can be generated for one type of polyfunctional secondary thiol compound (A) depending on the combination of mercapto groups forming disulfide bonds. The disulfide dimers (B) may be any of these multiple types of compounds. That is, in the above-described chemical formula RSSR, the two R's may have different structures derived from the same molecule.

In the thiol-containing composition of the present embodiment, since the disulfide dimers (B) are disulfide dimers (B) of polyfunctional secondary thiol compounds (A) contained in the thiol-containing composition, the molecular structures of the disulfide dimers (B) are similar to the molecular structures of the polyfunctional secondary thiol compounds (A) contained in the thiol-containing composition. For this reason, the disulfide dimers (B) are likely to be dissolved in the polyfunctional secondary thiol compounds (A). In addition, the incorporation of the disulfide dimers (B) in the thiol-containing composition has little effect on the features and performance of the thiol-containing composition, and features and performance due to incorporation of the polyfunctional secondary thiol compounds (A) are likely to be exhibited.

In a case where there are two or more types of polyfunctional secondary thiol compounds (A) in the thiol-containing composition, the disulfide dimers (B) may include disulfide dimers (B) of one or more types of polyfunctional secondary thiol compounds (A) in the two or more types of polyfunctional secondary thiol compounds (A).

A disulfide dimer (B) is a compound shown below in a case where, for example, a polyfunctional secondary thiol compound (A) is 3-mercaptobutanoic acid ester represented by any of Formulae (1) to (6) above.

In a case where the polyfunctional secondary thiol compound (A) is 3-mercaptobutanoic acid ester represented by Formula (1) above, the disulfide dimer (B) is a compound represented by Formula (7) below. In a case where the polyfunctional secondary thiol compound (A) is 3-mercaptobutanoic acid ester represented by Formula (2) above, the disulfide dimer (B) is a compound represented by Formula (8) below. In a case where the polyfunctional secondary thiol compound (A) is 3-mercaptobutanoic acid ester represented by Formula (3) above, the disulfide dimer (B) is a compound represented by Formula (9) below. In a case where the polyfunctional secondary thiol compound (A) is 3-mercaptobutanoic acid ester represented by Formula (4) above, the disulfide dimer (B) is a compound represented by Formula (10) below.

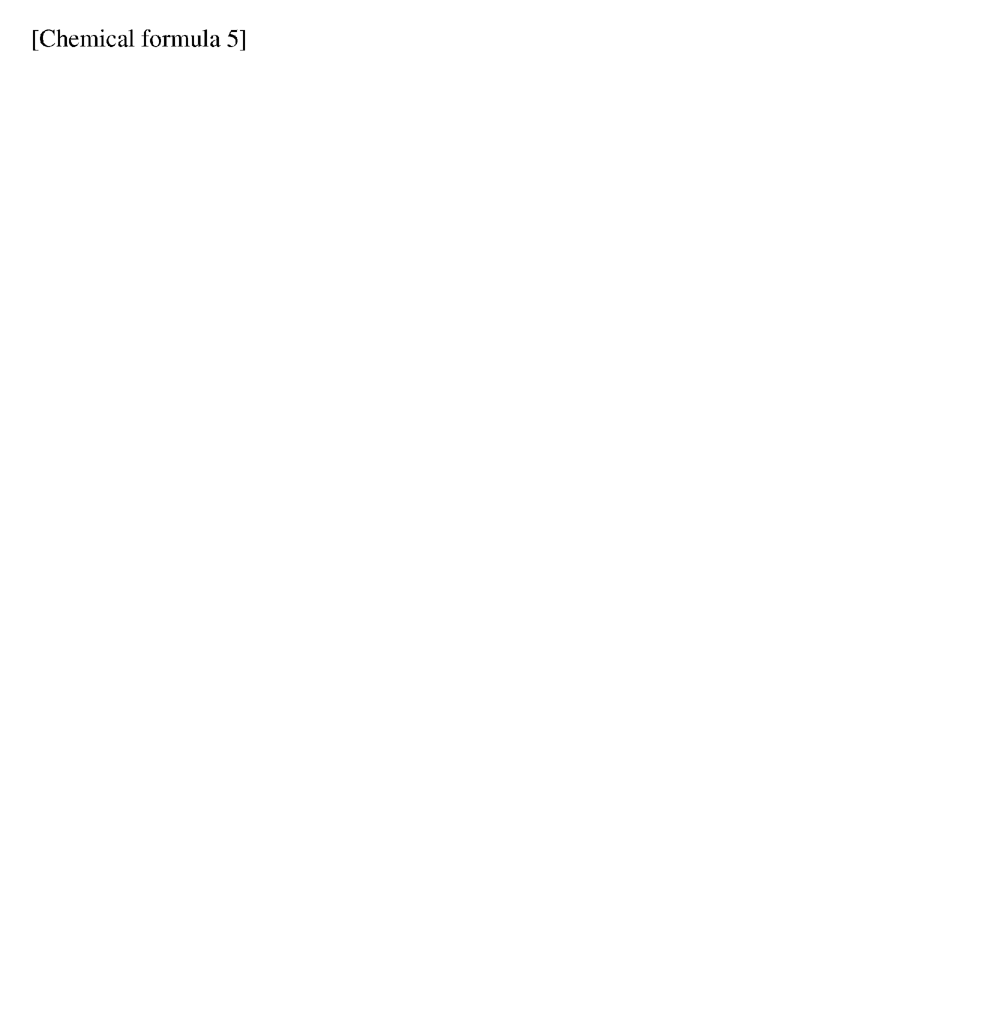

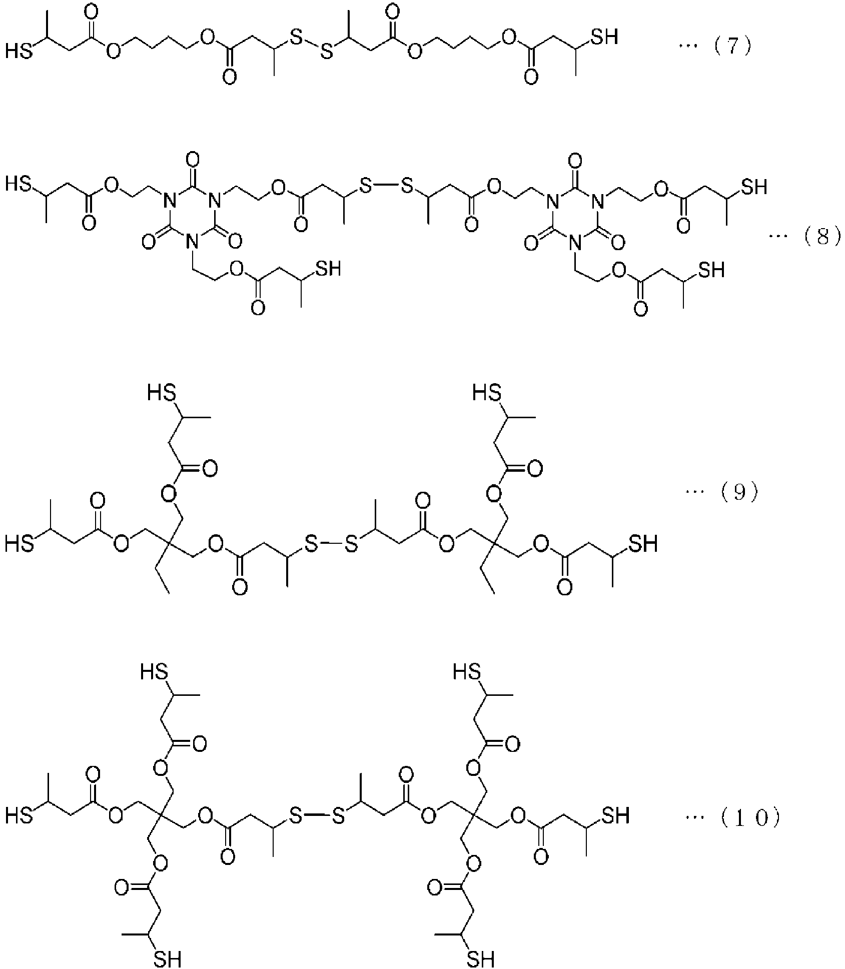

In a case where the polyfunctional secondary thiol compound (A) is 3-mercaptobutanoic acid ester represented by General Formula (5) above, the disulfide dimer (B) is a compound represented by General Formula (11) below. 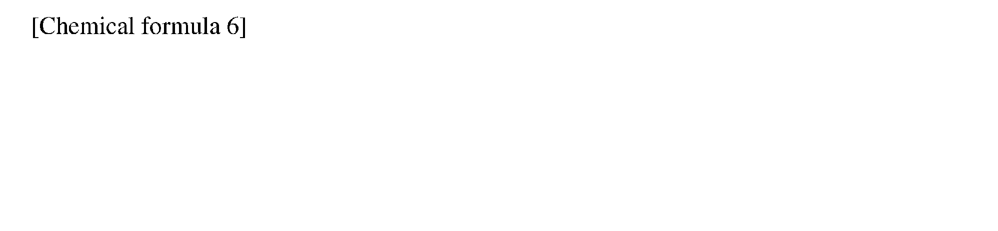 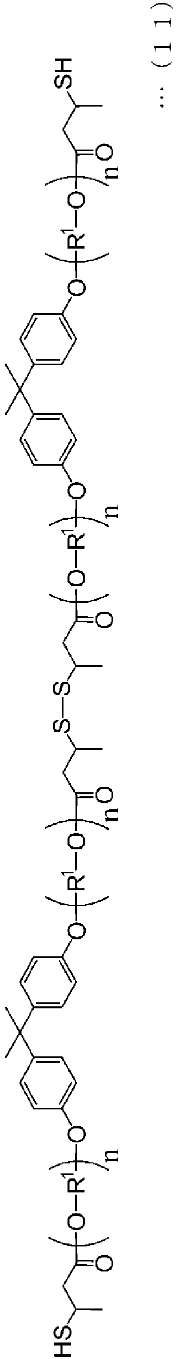 (In Formula (11), R¹ and n are the same as those in Formula (5).)

In a case where the polyfunctional secondary thiol compound (A) is 3-mercaptobutanoic acid ester represented by General Formula (6) above, the disulfide dimer (B) is a compound represented by General Formula (12) below. 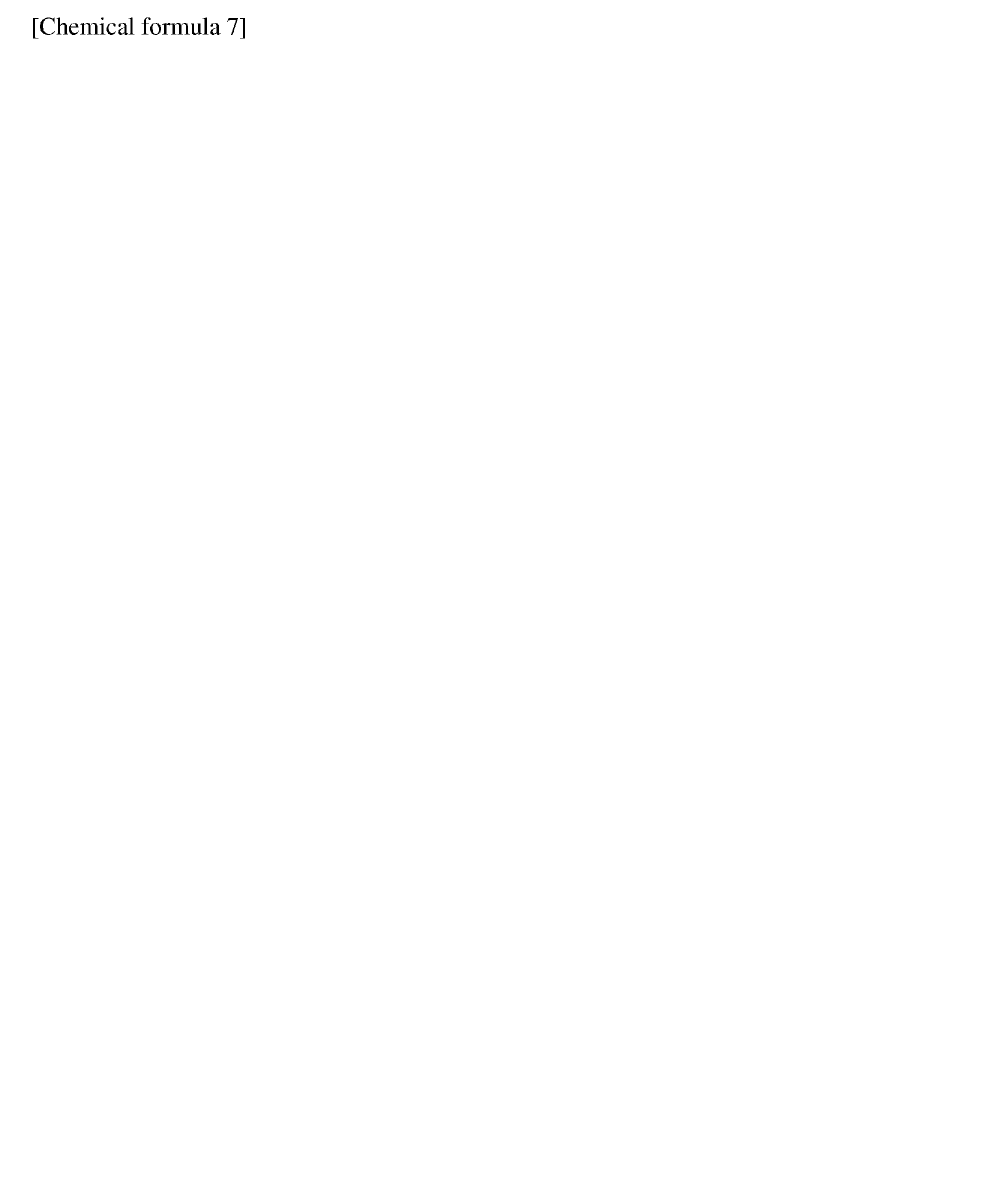 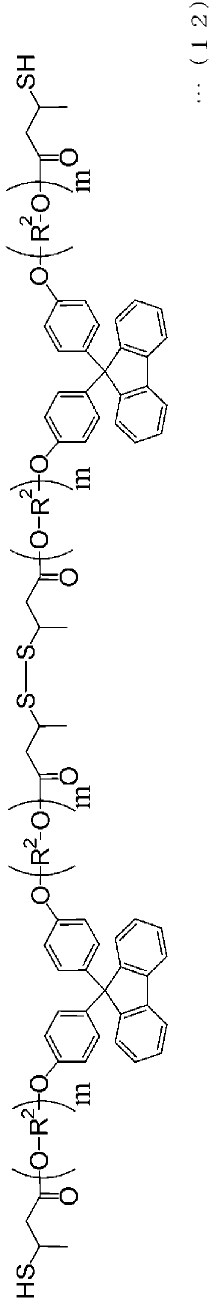 (In Formula (12), R² and m are the same as those in Formula (6).)

Disulfide dimers (B) can be synthesized through a well-known conventional method.

For example, it can be produced through a method of reacting polyfunctional secondary thiol compounds (A) with an oxidizer in a solvent.

Specifically, it can be synthesized through the methods disclosed in Non-Patent Documents 1 and 2 in which the polyfunctional secondary thiol compounds (A) are reacted with iodine in an alcohol solvent such as methanol.

In the method of reacting the polyfunctional secondary thiol compounds (A) with an oxidizer in a solvent, it is preferable to use a method of adding a solution (second dilute solution) of an oxidizer diluted with a solvent to a solution (first dilute solution) of the polyfunctional secondary thiol compounds (A) diluted in a solvent. In this case, since the concentration of the polyfunctional secondary thiol compounds (A) and the oxidizer in the reaction solution is low, trimer or higher disulfide polymers are less likely to be generated, and disulfide dimers (B) can be selectively and efficiently produced. The concentration of the polyfunctional secondary thiol compounds (A) in the first dilute solution is preferably 10 mass% or less, more preferably 1 mass% or less. The concentration of the oxidizer in the second dilute solution is preferably 10 mass% or less, more preferably 1 mass% or less.

When adding the second dilute solution to the first dilute solution, it is preferable to add the second dilute solution little by little while stirring the first dilute solution. As a result, it is possible to suppress formation of a region with a locally high concentration of the second dilute solution in the mixed solution of the first dilute solution and the second dilute solution and to effectively suppress generation of the trimer or higher disulfide polymers. As stirring means for stirring the first dilute solution and the second dilute solution, for example, a magnetic stirrer can be preferably used. The amount of the first dilute solution and second dilute solution supplied is preferably 1 mL/min or less, more preferably 0.1 mL/min or less.

The amount of the disulfide dimers (B) contained in the thiol-containing composition of the present embodiment is 0.001 to 1.5 parts by mass based on 100 parts by mass of the polyfunctional secondary thiol compounds (A), and can be appropriately determined depending on the types of polyfunctional secondary thiol compounds (A), the features required for the thiol-containing composition. In a case where there are multiple types of disulfide dimers (B) for one type of polyfunctional secondary thiol compound (A) (that is, the polyfunctional secondary thiol compound (A) has an asymmetric structure), the amount of the disulfide dimers (B) is the total amount of these multiple types of compounds.

In a case where the thiol-containing composition of the present embodiment contains two or more types of polyfunctional secondary thiols (A), the amount of disulfide dimers (B) corresponding to the polyfunctional secondary thiols (A) may be 0.001 to 1.5 parts by mass based on 100 parts by mass of at least one type of polyfunctional secondary thiol (A) contained in the thiol-containing composition.

If the amount of the disulfide dimers (B) is 0.001 parts by mass or more based on 100 parts by mass of the polyfunctional secondary thiol compounds (A), an effect of improving storage stability of the thiol-containing composition and curable compositions (a photosetting composition and a thermosetting composition) containing this thiol-containing composition can be sufficiently obtained. The amount of the disulfide dimers (B) is preferably 0.005 parts by mass or more based on 100 parts by mass of the polyfunctional secondary thiol compounds (A) to further improve the storage stability of the thiol-containing composition.

If the amount of the disulfide dimers (B) is 1.5 parts by mass or less based on 100 parts by mass of the polyfunctional secondary thiol compounds (A), the deviation between the features and performance of the thiol-containing composition and the features and performance of the polyfunctional secondary thiol compounds (A) is sufficiently small. As a result, the thiol-containing composition of the present embodiment and the curable compositions containing this have sufficient features and performance due to incorporation of the polyfunctional secondary thiol compounds (A). The amount of the disulfide dimers (B) is preferably 1.0 parts by mass or less, more preferably 0.5 parts by mass or less based on 100 parts by mass of the polyfunctional secondary thiol compounds (A). The amount of the disulfide dimers (B) may be selected from the above-described ranges as necessary. For example, the amount of the disulfide dimers (B) may be, based on 100 parts by mass of the polyfunctional secondary thiol compounds (A), 0.0010 to 1.50 parts by mass, 0.001 to 1.30 parts by mass, 0.002 to 0.80 parts by mass, 0.004 to 0.60 parts by mass, 0.008 to 0.40 parts by mass, 0.010 to 0.20 parts by mass, or 0.012 to 0.18 parts by mass. However, the present invention is not limited to these examples.

The thiol-containing composition of the present embodiment may contain other components in addition to the polyfunctional secondary thiols (A) and the disulfide dimers (B) of the polyfunctional secondary thiols (A).

Examples of other components include a polyfunctional secondary thiol that is not the polyfunctional secondary thiol (A), a disulfide that is not the disulfide dimer (B) of polyfunctional secondary thiols (A), and impurities such as a byproduct produced during synthesis of the polyfunctional secondary thiols (A) and/or synthesis of the disulfide dimer (B).

The thiol-containing composition of the present embodiment may contain trimer or higher disulfide polymers formed from three or more molecules of the same polyfunctional secondary thiol compounds (A) contained in the thiol-containing composition.

The amount of the polyfunctional secondary thiols (A) contained in the thiol-containing composition of the present embodiment is preferably 70 mass% or more, more preferably 80 mass% or more, and still more preferably 90 mass% or more. The upper limit value of the polyfunctional secondary thiols (A) can be arbitrarily selected. For example, 99.999 mass% or less, 99.9 mass% or less, 99.5 mass% or less, 99.0 mass% or less, 98.0 mass% or less, 95.0 mass% or less, 93.0 mass% or less, 90.0 mass% or less, 85.0 mass% or less, or 80.0 mass% or less. However, the present invention is not limited to these examples.

In a case where the thiol-containing composition contains two or more types of polyfunctional secondary thiols (A), the proportion of disulfide dimers (B) having a molecular structure corresponding to a polyfunctional secondary thiol (A) with the largest content in the polyfunctional secondary thiols (A) preferably satisfies at least the above-described proportions. The proportion of the polyfunctional secondary thiols (A) satisfying the above-described proportions contained in the thiol-containing composition is preferably 70 mass% or higher, more preferably 80 mass% or higher, and still more preferably 90 mass% or higher.

### "Method for producing thiol-containing composition"

As a method for producing the thiol-containing composition of the present embodiment, it is possible to use, for example, a method of mixing the above-described polyfunctional secondary thiol compounds (A) with disulfide dimers (B) thereof.

Specifically, a method of adding a predetermined amount of disulfide dimers (B) synthesized separately from polyfunctional secondary thiol compounds (A) to the polyfunctional secondary thiol compounds (A) for mixing can be used.

In addition, as a method for producing a thiol-containing composition, the following production method may be used. That is, it is possible to use a method of reacting a predetermined amount of polyfunctional secondary thiol compounds (A) with a predetermined amount of oxidizer in a solvent to convert some polyfunctional secondary thiol compounds (A) into disulfide dimers and to produce a predetermined amount of disulfide dimers (B) of the polyfunctional secondary thiol compounds (A). As this method for producing a thiol-containing composition, it is possible to use the same method as that of reacting polyfunctional secondary thiol compounds (A) with an oxidizer in a solvent in the above-described method for producing disulfide dimers (B).

Accordingly, in this production method, an alcohol solvent such as methanol can be used as a solvent, for example. As the oxidizer, iodine or the like can be used, for example. In this production method, since the step of mixing polyfunctional secondary thiol compounds (A) with disulfide dimers (B) thereof is not performed, a thiol-containing composition can be more simply produced compared to the case of using the method of mixing polyfunctional secondary thiol compounds (A) with disulfide dimers (B) thereof.

In a case where a thiol-containing composition is produced through this method, an oxidizer and a reactant of the oxidizer may remain in a reaction product obtained by reacting the polyfunctional secondary thiol compounds (A) with the oxidizer. If the oxidizer and the reactant of the oxidizer remain in the thiol-containing composition, there is a concern that features of the thiol-containing composition and curable compositions containing this may be adversely affected. For this reason, after reacting polyfunctional secondary thiol compounds (A) with an oxidizer and converting some polyfunctional secondary thiol compounds (A) into disulfide dimers, the oxidizer and a reactant of the oxidizer are preferably removed from the resulting reaction product to obtain a thiol-containing composition.

The thiol-containing composition of the present embodiment includes: polyfunctional secondary thiol compounds (A) each of which is an ester of a polyhydric alcohol with a carboxylic acid having a secondary mercapto group; and disulfide dimers (B) of the polyfunctional secondary thiol compounds (A), in which the amount of the disulfide dimers (B) is 0.001 to 1.5 parts by mass based on 100 parts by mass of the polyfunctional secondary thiol compounds (A). For this reason, the thiol-containing composition of the present embodiment has sufficient features and performance due to incorporation of the polyfunctional secondary thiol compounds (A), and has superior storage stability compared to the polyfunctional secondary thiol compounds (A).

### [Photosetting composition]

A photosetting composition of the present embodiment contains: the above-described thiol-containing composition of the present embodiment; a compound having an ethylenically unsaturated group; and a photopolymerization initiator.

### "Compound having ethylenically unsaturated group"

As the compound having an ethylenically unsaturated group, a compound having an ethylenically unsaturated group that causes a radical polymerization (or crosslinking) reaction can be used. Specific examples of compounds having an ethylenically unsaturated group include various (meth)acrylic acid esters such as (meth)acrylic acid, methyl (meth)acrylate, butyl (meth)acrylate, benzyl (meth)acrylate, 2-hydroxyethyl (meth)acrylate, 2-hydroxypropyl (meth)acrylate, ethylene glycol di(meth)acrylate, 1,6-hexanediol di(meth)acrylate, pentaerythritol tri(meth)acrylate, styrene, divinylbenzene, (meth)acrylamide, vinyl acetate, N-hydroxymethyl (meth)acrylamide, dipentaerythritol hexaacrylate, melamine acrylate, and epoxy acrylate prepolymer. The term "(meth)acrylic" means both "methacrylic" and "acrylic."

As a compound having an ethylenically unsaturated group, a polyfunctional (meth)acrylic monomer is preferably used because it has favorable exposure sensitivity and various resistances after curing.

These compounds having an ethylenically unsaturated bond may be used alone or in combination of two or more thereof.

### "Photopolymerization initiator"

As a photopolymerization initiator, a general photopolymerization initiator can be used. Examples of preferred photopolymerization initiators include α-hydroxyacetophenones, α-aminoacetophenones, and biimidazoles.

Examples of α-hydroxyacetophenones include 2-hydroxy-2-methyl-1-phenylpropane-1-one, 2-hydroxy-2-methyl-1-phenylbutan-1-one, 1-(4-methylphenyl)-2-hydroxy-2-methylpropane-1-one, 1-(4-isopropylphenyl)-2-hydroxy-2-methylpropane-1-one, 1-(4-buthylphenyl)-2-hydroxy-2-methylpropane-1-one, 2-hydroxy-2-methyl-1-(4-octylphenyl)propane-1-one, 1-(4-dodecylphenyl)-2-hydroxy-2-methylpropane-1-one, 1-(4-methoxyphenyl)-2-hydroxy-2-methylpropane-1-one, 1-(4-methylthiophenyl)-2-hydroxy-2-methylpropane-1 -one, 1 -(4-chlorophenyl)-2-hydroxy-2-methylpropane-1 -one, 1 -(4-bromophenyl)-2-hydroxy-2-methylpropane-1 -one, 2-hydroxy-1-(4-hydroxyphenyl)-2-methylpropane-1-one, 1-(4-dimethylaminophenyl)-2-hydroxy-2-methylpropane-1-one, 1 -(4-carboethoxyphenyl)-2-hydroxy-2-methylpropane-1 -one, 1-hydroxycyclohexyl phenyl ketone, and 2-hydroxy-1-(4-(2-hydroxyethoxy)-phenyl)-2-methylpropane-1-one.

Examples of α-aminoacetophenones include 2-dimethylamino-2-methyl-1-phenylpropane-1-one, 2-diethylamino-2-methyl-1-phenylpropane-1-one, 2-methyl-2-morpholino-1-phenylpropane-1-one, 2-dimethylamino-2-methyl-1-(4-methylphenyl)propane-1 -one, 2-dmethylamino-1-(4-ethylphenyl)-2-methylpropane-1-one, 2-dmethylamino-1-(4-isopropylphenyl)-2-methylpropane-1-one, 1-(4-buthylphenyl)-2-dimethylamino-2-methylpropane-1-one, 2-dimethylamino-1 -(4-methoxyphenyl)-2-methylpropane-1-one, 2-dimethylamino-2-methyl-1-[4-(methylthio)phenyl]propane-1-one, 2-methyl-1-[4-(methylthio)phenyl]-2-morpholinopropane-1-one, 2-benzyl-2-dimethylamino-1-(4-morpholinophenyl)-butane-1-one, and 2-benzyl-2-dimethylamino-1-(4-dimethylaminophenyl)-butane-1-one.

Examples of biimidazoles include 2,2'-bis(2-chlorophenyl)-4,4',5,5'-tetraphenyl-1,2'-biimidazole, 2,2'-bis(2-chlorophenyl)-4,4',5,5'-tetra(ethoxyphenyl)-1,2'-biimidazole, 2,2'-bis(2-chlorophenyl)-4,4',5,5'-tetra(4-bromophenyl)-1,2'-biimidazole, 2,2'-bis(2-chlorophenyl)-4,4',5,5'-tetra(4-methylphenyl)-1,2'-biimidazole, 2,2'-bis(2-chlorophenyl)-4,4',5,5'-tetra(4-ethylphenyl)-1,2'-biimidazole, 2,2'-bis(2-chlorophenyl)-4,4',5,5'-tetra(4-buthylphenyl)-1,2'-biimidazole, 2,2'-bis(2-chlorophenyl)-4,4',5,5'-tetra(4-octylphenyl)-1,2'-biimidazole, 2,2'-bis(2-chlorophenyl)-4,4',5,5'-tetra(4-methoxyphenyl)-1,2'-biimidazole, 2,2'-bis(2-chlorophenyl)-4,4',5,5'-tetra(3-methoxyphenyl)-1,2'-biimidazole, 2,2'-bis(2,4-dichlorophenyl)-4,4',5,5'-tetraphenyl-1,2'-biimidazole, 2,2'-bis(2-chlorophenyl)-4,4',5,5'-tetra(3-methoxyphenyl)-1,2'-biimidazole, 2,2'-bis(2,6-dichlorophenyl)-4,4',5,5'-tetra(3-methoxyphenyl)-1,2'-biimidazole, and 2,2'-bis(2-methylphenyl)-4,4',5,5'-tetraphenyl-1,2'-biimidazole.

As photopolymerization initiators other than those described above, it is also possible to use benzoin methyl ether, benzoin ethyl ether, benzoin isopropyl ether, benzyl methyl ketal, α-halogenoacetophenones, methylphenyl glyoxylate, benzyl, anthraquinone, phenanthrene quinone, camphorquinone isophthalophenone, and acylphosphine oxide.

These photopolymerization initiators may be used singly or in combination of two or more thereof.

The amount of the photopolymerization initiator in the photosetting composition based on 100 parts by mass of the compound having an ethylenically unsaturated group is preferably 1 to 100 parts by mass, more preferably 2 to 50 parts by mass, and still more preferably 2 to 10 parts by mass. If the amount of the photopolymerization initiator is 1 part by mass or more, a photosetting composition that can undergo a photopolymerization initiation reaction is obtained. In addition, if the amount of the photopolymerization initiator is 100 parts by mass or less, the molecular weight of a cured product does not become too small, and a photosetting composition that is sufficiently cured is obtained.

The amount of a thiol-containing composition in the photosetting composition based on 100 parts by mass of the compound having an ethylenically unsaturated group is preferably 1 to 200 parts by mass, more preferably 10 to 100 parts by mass, and still more preferably 10 to 50 parts by mass. The amount of the thiol-containing composition in the photosetting composition based on 100 parts by mass of the compound having an ethylenically unsaturated group can be preferably set from the ranges. For example, it may be 5 to 180 parts by mass, 10 to 160 parts by mass, 20 to 140 parts by mass, 30 to 120 parts by mass, 40 to 110 parts by mass, 60 to 90 parts by mass, or 70 to 80 parts by mass. However, the present invention is not limited to these examples. If the amount of the thiol-containing composition is 1 part by mass or more, a photosetting composition that can undergo a photopolymerization initiation reaction is obtained. In addition, if the amount of the thiol-containing composition is 200 parts by mass or less, the molecular weight of a cured product does not become too small, and a photosetting composition that is sufficiently cured is obtained.

The photosetting composition of the present embodiment can be produced by mixing the thiol-containing composition of the present embodiment, a compound having an ethylenically unsaturated group, and a photopolymerization initiator through a well-known method.

The photosetting composition of the present embodiment contains the thiol-containing composition of the present embodiment. For this reason, the features and performance due to incorporation of the polyfunctional secondary thiol compounds (A) can be sufficiently obtained. Moreover, the photosetting composition of the present embodiment has superior storage stability compared to a case where the polyfunctional secondary thiol compounds (A) is used instead of the thiol-containing composition of the present embodiment.

### [Thermosetting composition]

A thermosetting composition of the present embodiment contains: the above-described thiol-containing composition of the present embodiment; a compound having an epoxy group; and a curing catalyst.

### "Compound having epoxy group"

As the compound having an epoxy group, a compound having an epoxy group that causes an epoxy ring-opening addition reaction with a nucleophilic agent can be used. Specific examples thereof include epoxy resins obtained by adding epichlorohydrin to polyhydric phenols such as bisphenol A, halogenated bisphenol A, bisphenol F, halogenated bisphenol F, resorcinol, hydroquinone, pyrocatechol, 4,4'-dihydroxybiphenyl, and 1,5-hydroxynaphthalene, polyhydric alcohols such as ethylene glycol, propylene glycol and glycerin, and aromatic dicarboxylic acids such as oxybenzoic acid and phthalic acid.

Among these compounds having an epoxy group, a compound having two or more epoxy groups in the molecule is preferably used from the viewpoint of sufficiently advancing curing of the thermosetting composition.

These compounds having an epoxy group may be used alone or in combination of two or more thereof.

### "Curing catalyst"

As the curing catalyst, a basic compound can be used. Specific examples thereof include trimethylamine, triethylamine, tetraethylmethylenediamine, tetramethylpropane-1,3-diamine, tetramethylhexane-1,6-diamine, pentamethyldiethylenetriamine, pentamethyldipropylenetriamine, bis(2-dimethylaminoethyl) ether, ethylene glycol (3-dimethyl) aminopropyl ether, dimethylaminoethanol, dimethylaminoethoxyethanol, N,N,N'-trimethylaminoethylethanolamine, dimethylcyclohexylamine, N,N-dimethylaminomethylphenol, N,N-dimethylpropylamine, N,N,N',N'-tetramethylhexamethylenediamine, N-methylpiperidine, N,N'-dimethylpiperazine, N,N-dimethylbenzylamine, dimethylaminomethylphenol, 2,4,6-tris(dimethylaminomethyl)phenol, 1,8-diazabicyclo[5.4.0]undecene-7, 1,5-diazabicyclo[4.3.0]-nonene-5,6-dibutylamino-1,8-diazabicyclo[5.4.0]-undecene-7, 1,2-dimethylimidazole, dimethylpiperazine, N-methyl-N'-(2-methylamino)-ethylpiperazine, N-methylmorpholine, N-(N',N'-dimethylamino)ethyl)morpholine, N-methyl-N'-(2-hydroxyethyl)morpholine, triethylenediamine, hexamethylenetetramine, ethylphosphine, phenylphosphine, dimethylphosphine, diphenylphosphine, trimethylphosphine, triethylphosphine, tributylphosphine, triphenylphosphine, tris(p-tolyl)phosphine, tris(alkylphenyl)phosphine, tris(alkoxyphenyl)phosphine, trimethyl phosphate, triethyl phosphate, triphenyl phosphate, trialkyl phosphate, tetraphenylphosphonium tetraphenylborate, and 1,4-bis(diphenylphosphino)butane.

Examples of commercially available curing catalysts include jER Cure (trademark registered) 3010 manufactured by Mitsubishi Chemical Corporation, Curezol 2PZ, 2PHZ, 2P4MHZ, C17Z, 2MZ-A, 2E4MZ-CNS, and 2MA-OK, which are imidazole compounds manufactured by Shikoku Chemicals Corporation, Amicure (registered trademark) PN-23, PN-40, PN-50, PN-H, MY-24, and MY-25 which are epoxy resin-amine adducts manufactured by Ajinomoto Fine-Techno Co., Inc., Adeka Hardener EH-3293S, EH-3366S, EH-3615S, EH-4070S, EH-4342S, and EH-3731S manufactured by Adeka Corporation, Novacure HX-3742 and HX-3721 which are epoxy resin-amine adducts manufactured by Asahi Kasei Corporation, and Fujicure FXR-1030, FXR-1081, and FXR-1110 which are modified aliphatic polyamines manufactured by T&K Toka Co., Ltd.

Among these, latent curing catalysts such as Curezol 2MZ-A, Amicure (registered trademark), Novacure, and Fujicure are preferable because they can provide a thermosetting composition having excellent storage stability.

These curing catalysts may be used singly or in combination of two or more thereof.

The amount of the curing catalyst in the thermosetting composition based on 100 parts by mass of the compound having an epoxy group is preferably 0.05 to 10 parts by mass, more preferably 0.1 to 5 parts by mass, and still more preferably 0.1 to 2 parts by mass. If the amount of the curing catalyst is 0.05 parts by mass or more, a polymerization reaction of the thermosetting composition will not progress too slowly of a high temperature will not be required to cause a polymerization reaction. In addition, when the amount of the curing catalyst is 10 parts by mass or less, the storage stability of the thermosetting composition will be even more favorable.

The amount of the thiol-containing composition in the thermosetting composition based on 100 parts by mass of the compound having an epoxy group is preferably 1 to 200 parts by mass, more preferably 5 to 100 parts by mass, and still more preferably 50 to 100 parts by mass. The amount of the thiol-containing composition in the thermosetting composition based on 100 parts by mass of the compound having an epoxy group can be preferably set from the ranges. For example, it may be 5 to 180 parts by mass, 10 to 160 parts by mass, 20 to 140 parts by mass, 30 to 120 parts by mass, 40 to 110 parts by mass, 60 to 90 parts by mass, or 70 to 80 parts by mass. However, the present invention is not limited to these examples. If the amount of the thiol-containing composition is 1 part by mass or more, insufficient curing due to unreacted epoxy groups remaining in cured products will not occur and the durability of cured products will not deteriorate. In addition, if the amount of the thiol-containing composition is 200 parts by mass or less, insufficient curing due to unreacted mercapto groups remaining in cured products will not occur and the durability of cured products will not deteriorate.

The thermosetting composition of the present embodiment can be produced by mixing the thiol-containing composition of the present embodiment, the compound having an epoxy group, and the curing catalyst through a well-known method.

The thermosetting composition of the present embodiment contains the thiol-containing composition of the present embodiment. For this reason, the features and performance due to incorporation of the polyfunctional secondary thiol compounds (A) can be sufficiently obtained. Moreover, the thermosetting composition of the present embodiment has superior storage stability compared to a case where the polyfunctional secondary thiol compounds (A) is used instead of the thiol-containing composition of the present embodiment.

In the thiol-containing composition, photosetting composition, and thermosetting composition of the present embodiment, the expression "excellent storage stability" means that the change in property that serves as an indicator of stability before and after storage when they are stored under certain conditions is small or that it takes a long time for the change in property that serves as an indicator of stability to reach a certain amount.

As properties that serve as indicators of stability of the thiol-containing composition, it is possible to use, for example, the amount of the polyfunctional secondary thiol compounds (A) contained in the thiol-containing composition, and physical properties such as the viscosity, odor, and color of the thiol-containing composition.

As properties that serve as indicators of stability of the curable compositions (the photosetting composition and the thermosetting composition), it is possible to use, for example, the amount of unreacted monomers contained in the curable compositions and properties such as the curing time and curing calorific value in addition to properties such as the viscosity and color of the curable compositions.

### [Examples]

Hereinafter, the present invention will be more specifically described using examples and comparative examples. The examples shown below are provided to facilitate understanding of the contents of the present invention. The present invention is not limited only to these examples.

The raw materials used when producing thiol-containing compositions, photosetting compositions, and thermosetting compositions of the examples and comparative examples shown below are as follows.
· Iodine (reagent special grade; manufactured by Junsei Chemical Co., Ltd.)
· Methanol (reagent special grade; manufactured by Junsei Chemical Co., Ltd.)
· Toluene (reagent special grade; manufactured by Junsei Chemical Co., Ltd.)
· Epoxy resin (compound having epoxy group): jER (trademark registered) 828 (manufactured by Mitsubishi Chemical Corporation)
· Curing catalyst: Amicure (registered trademark) PN-23 (manufactured by Ajinomoto Fine-Techno Co., Inc.) (adduct of imidazole and bisphenol A-type epoxy resin)
. Acrylic monomer (compound having ethylenically unsaturated group): 1,6-hexanediol dimethacrylate (manufactured by Tokyo Chemical Industry Co., Ltd.)
· Photopolymerization initiator: 1-hydroxycyclohexyl phenyl ketone

### (manufactured by Tokyo Chemical Industry Co., Ltd.)

In the examples and comparative examples shown below, storage stability of the thiol-containing compositions, photosetting compositions, and thermosetting compositions was evaluated using the methods shown below. The storage stability of raw material composition containing polyfunctional secondary thiol compounds (A) were evaluated in the same manner as storage stability of the thiol-containing compositions.

For each composition, a polypropylene container (I-Boy (registered trademark), 50 mL wide-mouth bottle, manufactured by As ONE Corporation) with a lid was used as a storage container for storing a test piece for evaluating the storage stability.

When evaluating the storage stability of each of the thiol-containing compositions and thermosetting compositions, a white LED (LED Base Light TENQOO (trade name); manufactured by Toshiba Lighting & Technology Corporation) was used as a light source during storage to irradiate the test piece in the storage container.

### (Storage stability of thiol-containing composition)

The storage stability of a thiol-containing composition was evaluated such that the content (parts by mass) of polyfunctional secondary thiol compounds (A) in 100 parts by mass of the thiol-containing composition immediately after synthesis and the content (parts by mass) of the polyfunctional secondary thiol compounds (A) in 100 parts by mass of the thiol-containing composition after storage at 60°C for 90 days while emitting light after synthesis were measured, and the maintenance rate of the polyfunctional secondary thiol compounds (A) immediately after synthesis and after storage was calculated by the following equation. [Maintenance rate] (%) = ([content after storage] / [content immediately after synthesis]) × 100

The contents (parts by mass) of the polyfunctional secondary thiol compounds (A) and disulfide dimers (B) in the thiol-containing composition were determined through high-performance liquid chromatography analysis. The analysis conditions were as follows.
Column: Shodex (registered trademark) 5C84E (manufactured by Showa Denko K.K.)
Eluent composition: Acetonitrile/water=3/1 (volume ratio) 2 mM tetra-n-butyl ammonium perchlorate
Pump: LC-10AD (manufactured by Shimadzu Corporation)
Eluent flow rate: 1.0 mL/minute
Temperature: 40°C
Detector: UV detector SPD-M10AVP (manufactured by Shimadzu Corporation)
Detection wavelength: 210 nm

### (Storage stability of thermosetting composition)

The storage stability of a thermosetting composition was evaluated such that the heat-curing calorific value of the thermosetting composition immediately after synthesis and the heat-curing calorific value of the thermosetting composition after storage at 40°C for 10 days while emitting light after synthesis were measured, and the maintenance rate of the heat-curing calorific values immediately after synthesis and after storage was calculated by the following equation. [Maintenance rate] (%) = ([heat-curing calorific value after storage] / [heat- curing calorific value imediately after synthesis]) × 100

The heat-curing calorific values were determined through differential scanning calorimetry (DSC). The analysis conditions were as follows.
Device: DSC7000 (manufactured by Hitachi High-Tech Corporation)
Atmosphere: Nitrogen gas
Temperature: Raised from 40°C to 200°C at rate of temperature increase of 10°C/min

### (Storage stability of photosetting composition)

The storage stability of a photosetting composition was evaluated such that the photocuring calorific value of the photosetting composition immediately after synthesis and the photocuring calorific value of the photosetting composition after storage at 60°C for 30 days in a dark place after synthesis were determined, and the maintenance rate of the photocuring calorific values immediately after synthesis and after storage was calculated. [Maintenance rate] (%) = ([photocuring calorific value after storage] / [photocuring calorific value immediately after synthesis]) × 100

The photocuring calorific values were determined through differential scanning calorimetry (DSC) under light irradiation. The analysis conditions were as follows.
Device: DSC7000 (manufactured by Hitachi High-Tech Corporation)
Atmosphere: Air
Temperature: Constant 25°C
Light irradiation amount: 20 mW/cm², 3 minutes

### [Example 1]

A raw material composition (raw material composition 1) containing polyfunctional secondary thiol compounds (A) represented by Formula (1) was synthesized according to the production method disclosed in Patent Document 5. After dissolving 20 g of the raw material composition 1 in 200 mL of methanol in a 500 mL flask, 0.2 g of a 1 mass% iodine methanol solution was added thereto little by little over 5 minutes while stirring with a magnetic stirrer to cause a reaction at room temperature for 3 hours. Methanol was removed from the reaction product using a rotary evaporator, the residue was dissolved in 40 mL of toluene, 20 mL of pure water was added thereto for mixing, and the mixture was subjected to liquid separation to obtain a toluene solution layer. Toluene was removed from this toluene solution using the rotary evaporator to obtain 20 g of a thiol-containing composition (thiol-containing composition 1) containing the polyfunctional secondary thiol compounds (A) represented by Formula (1) and disulfide dimers (B) represented by Formula (7).

The amount of the disulfide dimers (B) represented by Formula (7) in the thiol-containing composition 1 was measured through the above-described method. As a result, the amount of the disulfide dimers (B) in the thiol-containing composition 1 was 0.0106 parts by mass based on 100 parts by mass of the polyfunctional secondary thiol compounds (A) represented by Formula (1).

In addition, the storage stability of the thiol-containing composition 1 was evaluated through the above-described method. As a result, the maintenance rate of the polyfunctional secondary thiol compounds (A) immediately after synthesis and after storage was 99.2%.

7.8 g of the thiol-containing composition 1, 10 g of an epoxy resin, and 0.1 g of a curing catalyst were mixed with each other to prepare a thermosetting composition of Example 1. Then, the storage stability of the thermosetting composition of Example 1 was evaluated through the above-described method. As a result, the maintenance rate of the heat-curing calorific values immediately after synthesis and after storage was 91.6%.

1.9 g of the thiol-containing composition 1, 8.2 g of an acrylic monomer, and 0.2 g of a photopolymerization initiator were mixed with each other to prepare a photosetting composition of Example 1. Then, the storage stability of the photosetting composition of Example 1 was evaluated through the above-described method. As a result, the maintenance rate of the photocuring calorific values immediately after synthesis and after storage was 98.0%.

### [Comparative Example 1]

In Comparative Example 1, the above-described raw material composition 1 containing the polyfunctional secondary thiol compounds (A) represented by Formula (1) was used instead of the thiol-containing composition 1. The storage stability of the raw material composition 1 was evaluated through the above-described method. As a result, the maintenance rate of the polyfunctional secondary thiol compounds (A) immediately after synthesis and after storage was 97.2%.

In addition, a thermosetting composition and a photosetting composition of Comparative Example 1 were prepared in the same manner as in Example 1 except that the raw material composition 1 was used instead of the thiol-containing composition 1.

Then, the storage stability of the thermosetting composition and photosetting composition of Comparative Example 1 was evaluated through the above-described method. As a result, the maintenance rate of heat-curing calorific values immediately after synthesis of the thermosetting composition and after storage of the thermosetting composition was 79.4%, and the maintenance rate of the photocuring calorific values immediately after synthesis of the photosetting composition and after storage of the photosetting composition was 87.2%.

### [Example 2]

A raw material composition (raw material composition 2) containing polyfunctional secondary thiol compounds represented by Formula (2) was synthesized according to the method disclosed in Patent Document 5.

A thiol-containing composition (thiol-containing composition 2) containing the polyfunctional secondary thiol compounds (A) represented by Formula (2) and disulfide dimers (B) represented by Formula (8) was obtained in the same manner as in Example 1 except that the synthesized raw material composition 2 was used as a raw material composition containing the polyfunctional secondary thiol compounds (A).

The amount of the disulfide dimers (B) represented by Formula (8) in the thiol-containing composition 2 was measured through the above-described method. As a result, the amount of the disulfide dimers (B) in the thiol-containing composition 2 was 0.013 parts by mass based on 100 parts by mass of the polyfunctional secondary thiol compounds (A) represented by Formula (2).

In addition, the storage stability of the thiol-containing composition 2 was evaluated through the above-described method. As a result, the maintenance rate of the polyfunctional secondary thiol compounds (A) immediately after synthesis and after storage was 99.6%.

10 g of the thiol-containing composition 2, 10 g of an epoxy resin, and 0.1 g of a curing catalyst were mixed with each other to prepare a thermosetting composition of Example 2. Then, the storage stability of the thermosetting composition of Example 2 was evaluated through the above-described method. As a result, the maintenance rate of the heat-curing calorific values immediately after synthesis and after storage was 98.2%.

2.5 g of the thiol-containing composition 2, 8.2 g of an acrylic monomer, and 0.2 g of a photopolymerization initiator were mixed with each other to prepare a photosetting composition of Example 2. Then, the storage stability of the photosetting composition of Example 2 was evaluated through the above-described method. As a result, the maintenance rate of the photocuring calorific values immediately after synthesis and after storage was 98.7%.

### [Comparative Example 2]

In Comparative Example 2, the above-described raw material composition 2 containing the polyfunctional secondary thiol compounds (A) represented by Formula (2) was used instead of the thiol-containing composition 2. The storage stability of the raw material composition 2 was evaluated through the above-described method. As a result, the maintenance rate of the polyfunctional secondary thiol compounds (A) immediately after synthesis and after storage was 97.3%.

In addition, a thermosetting composition and a photosetting composition of Comparative Example 2 were prepared in the same manner as in Example 2 except that the raw material composition 2 was used instead of the thiol-containing composition 2.

Then, the storage stability of the thermosetting composition and photosetting composition of Comparative Example 2 was evaluated through the above-described method. As a result, the maintenance rate of heat-curing calorific values immediately after synthesis of the thermosetting composition and after storage of the thermosetting composition was 86.1%, and the maintenance rate of the photocuring calorific values immediately after synthesis of the photosetting composition and after storage of the photosetting composition was 92.3%.

### [Example 3]

A raw material composition (raw material composition 3) containing polyfunctional secondary thiol compounds represented by Formula (3) was synthesized according to the method disclosed in Patent Document 5.

A thiol-containing composition (thiol-containing composition 3) containing the polyfunctional secondary thiol compounds (A) represented by Formula (3) and disulfide dimers (B) represented by Formula (9) was obtained in the same manner as in Example 1 except that the synthesized raw material composition 3 was used as a raw material composition containing the polyfunctional secondary thiol compounds (A).

The amount of the disulfide dimers (B) represented by Formula (9) in the thiol-containing composition 3 was measured through the above-described method. As a result, the amount of the disulfide dimers (B) in the thiol-containing composition 3 was 0.0103 parts by mass based on 100 parts by mass of the polyfunctional secondary thiol compounds (A) represented by Formula (3).

In addition, the storage stability of the thiol-containing composition 3 was evaluated through the above-described method. As a result, the maintenance rate of the polyfunctional secondary thiol compounds (A) immediately after synthesis and after storage was 98.9%.

In addition, a thermosetting composition and a photosetting composition of Example 3 were prepared in the same manner as in Example 1 except that the thiol-containing composition 3 was used instead of the thiol-containing composition 1.

Then, the storage stability of the thermosetting composition and photosetting composition of Example 3 was evaluated through the above-described method. As a result, the maintenance rate of heat-curing calorific values immediately after synthesis of the thermosetting composition and after storage of the thermosetting composition was 95.9%, and the maintenance rate of the photocuring calorific values immediately after synthesis of the photosetting composition and after storage of the photosetting composition was 98.4%.

### [Comparative Example 3]

In Comparative Example 3, the above-described raw material composition 3 containing the polyfunctional secondary thiol compounds (A) represented by Formula (3) was used instead of the thiol-containing composition 3. The storage stability of the raw material composition 3 was evaluated through the above-described method. As a result, the maintenance rate of the polyfunctional secondary thiol compounds (A) immediately after synthesis and after storage was 96.2%.

In addition, a thermosetting composition and a photosetting composition of Comparative Example 3 were prepared in the same manner as in Example 3 except that the raw material composition 3 was used instead of the thiol-containing composition 3.

Then, the storage stability of the thermosetting composition and photosetting composition of Comparative Example 3 was evaluated through the above-described method. As a result, the maintenance rate of heat-curing calorific values immediately after synthesis of the thermosetting composition and after storage of the thermosetting composition was 82.2%, and the maintenance rate of the photocuring calorific values immediately after synthesis of the photosetting composition and after storage of the photosetting composition was 89.7%.

### [Example 4]

A raw material composition (raw material composition 4) containing polyfunctional secondary thiol compounds represented by Formula (4) was synthesized according to the method disclosed in Patent Document 5.

A thiol-containing composition (thiol-containing composition 4) containing the polyfunctional secondary thiol compounds (A) represented by Formula (4) and disulfide dimers (B) represented by Formula (10) was obtained in the same manner as in Example 1 except that the synthesized raw material composition 4 was used as a raw material composition containing the polyfunctional secondary thiol compounds (A).

The amount of the disulfide dimers (B) represented by Formula (10) in the thiol-containing composition 4 was measured through the above-described method. As a result, the amount of the disulfide dimers (B) in the thiol-containing composition 4 was 0.0098 parts by mass based on 100 parts by mass of the polyfunctional secondary thiol compounds (A) represented by Formula (4).

In addition, the storage stability of the thiol-containing composition 4 was evaluated through the above-described method. As a result, the maintenance rate of the polyfunctional secondary thiol compounds (A) immediately after synthesis and after storage was 99.2%.

7.2 g of the thiol-containing composition 4, 10 g of an epoxy resin, and 0.1 g of a curing catalyst were mixed with each other to prepare a thermosetting composition of Example 4. Then, the storage stability of the thermosetting composition of Example 4 was evaluated through the above-described method. As a result, the maintenance rate of the heat-curing calorific values immediately after synthesis and after storage was 97.9%.

1.8 g of the thiol-containing composition 4, 8.2 g of an acrylic monomer, and 0.2 g of a photopolymerization initiator were mixed with each other to prepare a photosetting composition of Example 4. Then, the storage stability of the photosetting composition of Example 4 was evaluated through the above-described method. As a result, the maintenance rate of the photocuring calorific values immediately after synthesis and after storage was 98.8%.

### [Comparative Example 4]

In Comparative Example 4, the above-described raw material composition 4 containing the polyfunctional secondary thiol compounds (A) represented by Formula (4) was used instead of the thiol-containing composition 4. The storage stability of the raw material composition 4 was evaluated through the above-described method. As a result, the maintenance rate of the polyfunctional secondary thiol compounds (A) immediately after synthesis and after storage was 95.4%.

In addition, a thermosetting composition and a photosetting composition of Comparative Example 4 were prepared in the same manner as in Example 4 except that the raw material composition 4 was used instead of the thiol-containing composition 4.

Then, the storage stability of the thermosetting composition and photosetting composition of Comparative Example 4 was evaluated through the above-described method. As a result, the maintenance rate of heat-curing calorific values immediately after synthesis of the thermosetting composition and after storage of the thermosetting composition was 84.2%, and the maintenance rate of the photocuring calorific values immediately after synthesis of the photosetting composition and after storage of the photosetting composition was 90.4%.

### [Example 5]

A thiol-containing composition (thiol-containing composition 5) of Example 5 was obtained in the same manner as in Example 4 except that 0.04 g of a 1 mass% iodine methanol solution was added over 1 minute.

The amount of the disulfide dimers (B) represented by Formula (10) in the thiol-containing composition 5 was measured through the above-described method. As a result, the amount of the disulfide dimers (B) in the thiol-containing composition 5 was 0.002 parts by mass based on 100 parts by mass of the polyfunctional secondary thiol compounds (A) represented by Formula (4).

In addition, the storage stability of the thiol-containing composition 5 was evaluated through the above-described method. As a result, the maintenance rate of the polyfunctional secondary thiol compounds (A) immediately after synthesis and after storage was 98.6%.

In addition, a thermosetting composition and a photosetting composition of Example 5 were prepared in the same manner as in Example 4 except that the thiol-containing composition 5 was used instead of the thiol-containing composition 4.

Then, the storage stability of the thermosetting composition and photosetting composition of Example 5 was evaluated through the above-described method. As a result, the maintenance rate of heat-curing calorific values immediately after synthesis of the thermosetting composition and after storage of the thermosetting composition was 93.6%, and the maintenance rate of the photocuring calorific values immediately after synthesis of the photosetting composition and after storage of the photosetting composition was 95.7%.

### [Comparative Example 5]

A thiol-containing composition (thiol-containing composition 5') of Comparative Example 5 was obtained in the same manner as in Example 4 except that 0.01 g of a 1 mass% iodine methanol solution was added over 1 minute.

The amount of the disulfide dimers (B) represented by Formula (10) in the thiol-containing composition 5' was measured through the above-described method. As a result, the amount of the disulfide dimers (B) in the thiol-containing composition 5' was 0.0004 parts by mass based on 100 parts by mass of the polyfunctional secondary thiol compounds (A) represented by Formula (4).

In addition, the storage stability of the thiol-containing composition 5' was evaluated through the above-described method. As a result, the maintenance rate of the polyfunctional secondary thiol compounds (A) immediately after synthesis and after storage was 96.7%.

In addition, a thermosetting composition and a photosetting composition of Comparative Example 5 were prepared in the same manner as in Example 4 except that the thiol-containing composition 5' was used instead of the thiol-containing composition 4. Then, the storage stability of the thermosetting composition and photosetting composition of Comparative Example 5 was evaluated through the above-described method. As a result, the maintenance rate of heat-curing calorific values immediately after synthesis of the thermosetting composition and after storage of the thermosetting composition was 84.8%, and the maintenance rate of the photocuring calorific values immediately after synthesis of the photosetting composition and after storage of the photosetting composition was 93.3%.

### [Example 6]

A thiol-containing composition (thiol-containing composition 6) of Example 6 was obtained in the same manner as in Example 4 except that 20 g of a 1 mass% iodine methanol solution was added over 40 minutes.

The amount of the disulfide dimers (B) represented by Formula (10) in the thiol-containing composition 6 was measured through the above-described method. As a result, the amount of the disulfide dimers (B) in the thiol-containing composition 6 was 0.93 parts by mass based on 100 parts by mass of the polyfunctional secondary thiol compounds (A) represented by Formula (4).

In addition, the storage stability of the thiol-containing composition 6 was evaluated through the above-described method. As a result, the maintenance rate of the polyfunctional secondary thiol compounds (A) immediately after synthesis and after storage was 99.4%.

In addition, a thermosetting composition and a photosetting composition of Example 6 were prepared in the same manner as in Example 4 except that the thiol-containing composition 6 was used instead of the thiol-containing composition 4.

Then, the storage stability of the thermosetting composition and photosetting composition of Example 6 was evaluated through the above-described method. As a result, the maintenance rate of heat-curing calorific values immediately after synthesis of the thermosetting composition and after storage of the thermosetting composition was 98.4%, and the maintenance rate of the photocuring calorific values immediately after synthesis of the photosetting composition and after storage of the photosetting composition was 99.6%.

### [Example 7]

A thiol-containing composition (thiol-containing composition 7) of Example 7 was obtained in the same manner as in Example 4 except that 30 g of a 1 mass% iodine methanol solution was added over 60 minutes.

The amount of the disulfide dimers (B) represented by Formula (10) in the thiol-containing composition 7 was measured through the above-described method. As a result, the amount of the disulfide dimers (B) in the thiol-containing composition 7 was 1.41 parts by mass based on 100 parts by mass of the polyfunctional secondary thiol compounds (A) represented by Formula (4).

In addition, the storage stability of the thiol-containing composition 7 was evaluated through the above-described method. As a result, the maintenance rate of the polyfunctional secondary thiol compounds (A) immediately after synthesis and after storage was 99.6%.

In addition, a thermosetting composition and a photosetting composition of Example 7 were prepared in the same manner as in Example 4 except that the thiol-containing composition 7 was used instead of the thiol-containing composition 4.

Then, the storage stability of the thermosetting composition and photosetting composition of Example 7 was evaluated through the above-described method. As a result, the maintenance rate of heat-curing calorific values immediately after synthesis of the thermosetting composition and after storage of the thermosetting composition was 99.0%, and the maintenance rate of the photocuring calorific values immediately after synthesis of the photosetting composition and after storage of the photosetting composition was 98.7%.

### [Example 8]

A raw material composition (raw material composition 1) containing polyfunctional secondary thiol compounds (A) represented by Formula (1) was synthesized in the same manner as in Example 1. After dissolving 10 g of the raw material composition 1 in 100 mL of methanol in a 200 mL flask, 10 g of a 10 mass% iodine methanol solution was added thereto little by little over 30 minutes while stirring with a magnetic stirrer to cause a reaction at room temperature for 4 hours. Methanol was removed from the reaction product using a rotary evaporator, the residue was dissolved in 20 mL of toluene, 10 mL of pure water was added thereto for mixing, and the mixture was subjected to liquid separation to obtain a toluene solution layer. Toluene was removed from this toluene solution using the rotary evaporator to obtain 9.8 g of a residue.

The entire amount of this residue was dissolved in 10 g of acetonitrile, fractionation through high-performance liquid chromatography was repeated 30 times, a total of 300 mL of a mixed solution of an eluent and the disulfide dimers (B) represented by Formula (7) was obtained.

The high-performance liquid chromatography device and fractionation conditions used for fractionating the disulfide dimers (B) are as follows.
Column: Shodex (registered trademark) 5C820E (manufactured by Showa Denko K.K.)
Eluent composition: Acetonitrile/water=3/1 (volume ratio), 0.1 wt% phosphoric acid
Pump: PU-2086 (manufactured by JASCO Corporation)
Eluent flow rate: 8.0 mL/minute
Temperature: 40°C
Detector: UV Detector UV-2075 (manufactured by JASCO Corporation)
Detection wavelength: 210 nm
Sample injection amount: 1.5 mL
Fraction collector: CHF122SC (manufactured by Advantech Co., Ltd.)

A 5% sodium hydroxide aqueous solution was added to a mixed liquid of an eluent and the disulfide dimers (B) represented by Formula (7) until the pH of the mixed liquid became 7.5. Thereafter, a solvent was removed from the mixed liquid using a rotary evaporator to obtain 1.1 g of a residue. This residue was dissolved in 3.0 mL of toluene, 1.5 mL of pure water was added thereto for mixing, and the mixture was subjected to liquid separation to obtain a toluene solution layer. Toluene was removed from this toluene solution using the rotary evaporator to obtain 1.0 g of a composition (raw material composition 8) containing the disulfide dimers (B) represented by Formula (7).

The amount of the disulfide dimers (B) represented by Formula (7) in the raw material composition 8 obtained in Example 8 was measured through the same method as the above-described method of measuring the amount of the disulfide dimers (B) in the thiol-containing composition. As a result, the amount of the disulfide dimers (B) represented by Formula (7) in the raw material composition 8 was 99.6 mass%.

20 g of the above-described raw material composition 1 containing the polyfunctional secondary thiol compounds (A) represented by Formula (1) was placed in a 100 mL flask, 0.2 g of the obtained raw material composition 8 obtained in Example 8 was further added thereto, the flask was stoppered with a plug, and the mixture was mixed well through shaking to obtain 20.2 g of a thiol-containing composition (thiol-containing composition 8) containing the polyfunctional secondary thiol compounds (A) represented by Formula (1) and the disulfide dimers (B) represented by Formula (7).

The amount of the disulfide dimers (B) represented by Formula (7) in the thiol-containing composition 8 was measured through the above-described method. As a result, the amount of the disulfide dimers (B) in the thiol-containing composition 8 was 0.0099 parts by mass based on 100 parts by mass of the polyfunctional secondary thiol compounds (A) represented by Formula (1).

In addition, the storage stability of the thiol-containing composition 8 was evaluated through the above-described method. As a result, the maintenance rate of the polyfunctional secondary thiol compounds (A) immediately after synthesis and after storage was 99.4%.

7.8 g of the thiol-containing composition 8, 10 g of an epoxy resin, and 0.1 g of a curing catalyst were mixed with each other to prepare a thermosetting composition of Example 8. Then, the storage stability of the thermosetting composition of Example 8 was evaluated through the above-described method. As a result, the maintenance rate of the heat-curing calorific values immediately after synthesis and after storage was 91.0%.

1.9 g of the thiol-containing composition 8, 8.2 g of an acrylic monomer, and 0.2 g of a photopolymerization initiator were mixed with each other to prepare a photosetting composition. Then, the storage stability of the photosetting composition of Example 8 was evaluated through the above-described method. As a result, the maintenance rate of the photocuring calorific values immediately after synthesis and after storage was 98.4%.

Chemical formulae of the disulfide dimers (B) and polyfunctional secondary thiol compounds (A) contained in the thiol-containing compositions of Examples 1 to 8 and Comparative Example 5, the amount of the polyfunctional secondary thiol compounds (A) based on 100 parts by mass of each thiol-containing composition, the amount of the disulfide dimers (B) based on 100 parts by mass of the polyfunctional secondary thiol compounds (A), and evaluation results of the storage stability of the thiol-containing compositions, thermosetting compositions, and photosetting compositions are shown in Table 1.

In addition, chemical formulae of polyfunctional secondary thiol compounds (A) contained in raw material compositions containing the polyfunctional secondary thiol compounds (A) of Comparative Examples 1 to 4, the amount of the polyfunctional secondary thiol compounds (A) based on 100 parts by mass of each raw material composition, and evaluation results of the storage stability of the raw material compositions, thermosetting compositions, and photosetting compositions are shown in Table 1. In Comparative Examples 1 to 4, the evaluation results of the storage stability of the raw material compositions were listed in the column of the evaluation results of the storage stability of the thiol-containing compositions in Table 1.

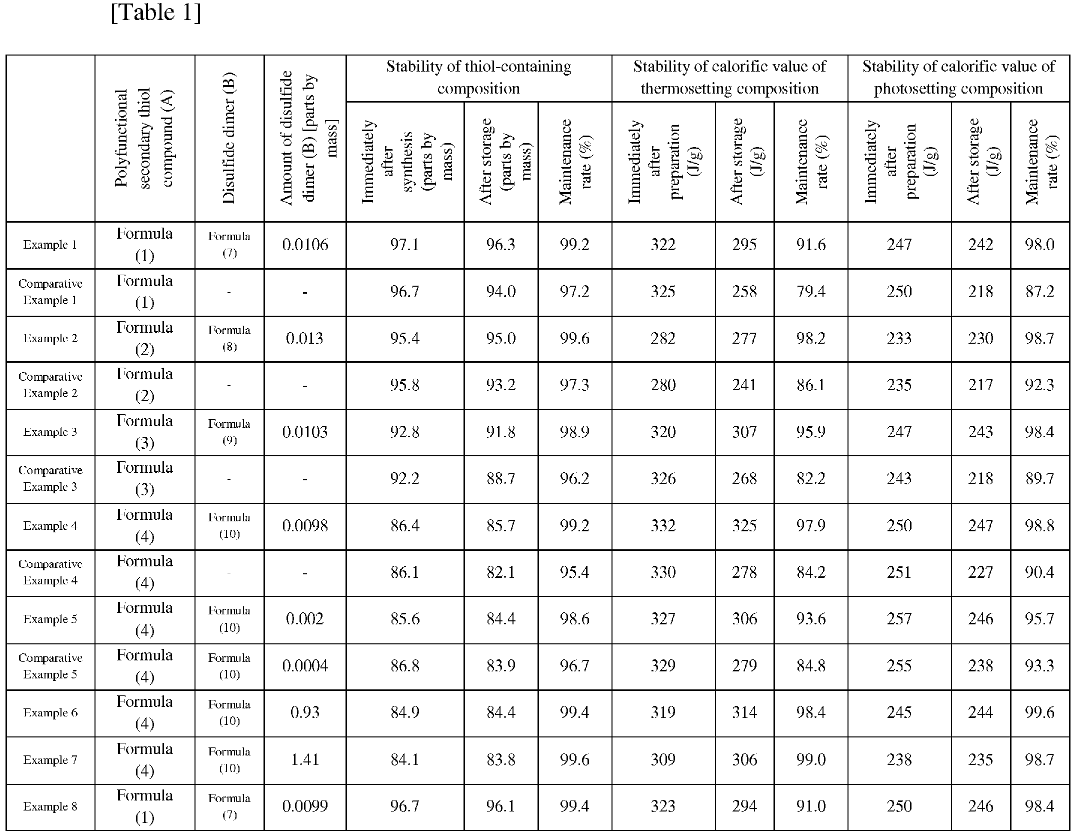

As shown in Table 1, it was possible to confirm that the thiol-containing compositions of Examples 1 to 8 containing polyfunctional secondary thiol compounds (A) and 0.001 to 1.5 parts by mass of disulfide dimers (B) based on 100 parts by mass of the polyfunctional secondary thiol compounds (A) had excellent storage stability and high maintenance rates of the polyfunctional secondary thiol compounds (A) immediately after synthesis and after storage compared to Comparative Examples 1 to 4 which were raw material compositions containing the polyfunctional secondary thiol compounds (A) respectively used as raw materials of Examples 1 to 8.

As shown in Table 1, it was possible to confirm that the thermosetting compositions and photosetting compositions containing the thiol-containing compositions of Examples 1 to 8 had excellent storage stability compared to the thermosetting compositions and photosetting compositions of Comparative Examples 1 to 4.

In addition, as shown in Table 1, it was possible to confirm that the thiol-containing compositions of Examples 4 to 7 had excellent storage stability and high maintenance rates of the polyfunctional secondary thiol compounds (A) immediately after synthesis and after storage compared to Comparative Example 5 in which the amount of the disulfide dimers (B) was less than 0.001 parts by mass.

In addition, as shown in Table 1, it was possible to confirm that the thermosetting compositions and photosetting compositions containing the thiol-containing compositions of Examples 4 to 7 had excellent storage stability compared to the thermosetting composition and photosetting composition of Comparative Example5.

In addition, as shown in Table 1, the heat-curing calorific value immediately after preparation of the thermosetting composition of Example 7 was 309 J/g which was lower than the heat-curing calorific value immediately after preparation of the thermosetting compositions of Examples 4 to 6 and Comparative Example 4. In addition, the photocuring calorific value immediately after preparation of the photosetting composition of Example 7 was 238 J/g which was lower than the heat-curing calorific value immediately after preparation of the photosetting compositions of Examples 4 to 6 and Comparative Example 4. From these facts, it was found that, in a case where the amount of disulfide dimers (B) in each thiol-containing composition is set to 1.0 part by mass or less based on 100 parts by mass of polyfunctional secondary thiol compounds (A), the deviation in curability between the case and a thermosetting composition and photosetting composition (refer to Comparative Example 4) containing the same polyfunctional secondary thiol compounds (A) and no disulfide dimers (B) tended to decrease.

In addition, as shown in Table 1, the thiol-containing composition of Example 8 and the thiol-containing composition of Example 1 had substantially the same evaluation results of the storage stability. That is, there was no difference in the storage stability of the thiol-containing compositions whether they were produced through a method of mixing polyfunctional secondary thiol compounds (A) with disulfide dimers (B) (Example 8) or through a method of reacting polyfunctional secondary thiol compounds (A) with an oxidizer in a solvent (Example 1). From this, it was possible to confirm that a thiol-containing composition containing disulfide dimers (B) exhibited excellent storage stability.

### [Industrial Applicability]

The present invention provides a thiol-containing composition having excellent storage stability, and an excellent photosetting composition and thermosetting composition containing the thiol-containing composition.

## Claims

1. A thiol-containing composition comprising:
polyfunctional secondary thiol compounds (A); and
disulfide dimers (B),
wherein the polyfunctional secondary thiol compounds (A) are each an ester of a polyhydric alcohol with a carboxylic acid having a secondary mercapto group,
wherein the disulfide dimers (B) are disulfide dimers of the polyfunctional secondary thiol compounds (A), and
wherein an amount of the disulfide dimers (B) is 0.001 to 1.5 parts by mass based on 100 parts by mass of the polyfunctional secondary thiol compounds (A).

2. The thiol-containing composition according to claim 1,
wherein an amount of the disulfide dimers (B) is 0.001 to 1.0 parts by mass based on 100 parts by mass of the polyfunctional secondary thiol compounds (A).

3. The thiol-containing composition according to claim 1 or 2,
wherein the polyfunctional secondary thiol compounds (A) are each an ester of 3-mercaptobutanoic acid with a polyhydric alcohol.

4. The thiol-containing composition according to any one of claims 1 to 3,
wherein the polyfunctional secondary thiol compounds (A) are at least one selected from Formulae (1) to (4) below.

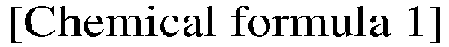

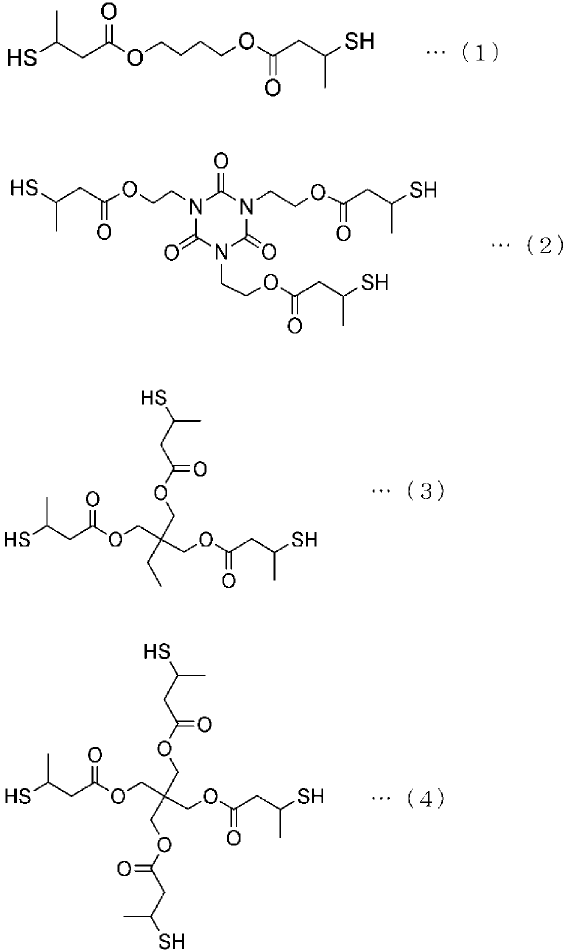

5. A photosetting composition comprising:
the thiol-containing composition according to any one of claims 1 to 4;
a compound having an ethylenically unsaturated group; and
a photopolymerization initiator.

6. The photosetting composition according to claim 5,
wherein an amount of the thiol-containing composition is 1 to 200 parts by mass based on 100 parts by mass of the compound having an ethylenically unsaturated group.

7. The photosetting composition according to claim 5 or 6,
wherein an amount of the photopolymerization initiator is 1 to 100 parts by mass based on 100 parts by mass of the compound having an ethylenically unsaturated group.

8. A thermosetting composition comprising:
the thiol-containing composition according to any one of claims 1 to 4;
a compound having an epoxy group; and
a curing catalyst.

9. The thermosetting composition according to claim 8,
wherein an amount of the thiol-containing composition is 1 to 200 parts by mass based on 100 parts by mass of the compound having an epoxy group.

10. The thermosetting composition according to claim 8 or 9,
wherein an amount of the curing catalyst is 0.05 to 10 parts by mass based on 100 parts by mass of the compound having an epoxy group.
